Europäisches Patentamt

European Patent Office

Office européen des brevets

(11) Veröffentlichungsnummer: **0 181 564**
**B1**

(12) **EUROPÄISCHE PATENTSCHRIFT**

(45) Veröffentlichungstag der Patentschrift: **16.05.90**

(21) Anmeldenummer: **85113806.5**

(22) Anmeldetag: **30.10.85**

(51) Int. Cl.$^5$: **A 61 K 31/19**, A 61 K 9/50

(54) Pharmazeutische Zubereitung mit einem Gehalt an Ibuprofen, sowie Verfahren zu ihrer Herstellung.

(30) Priorität: **05.11.84 DE 3440288**

(43) Veröffentlichungstag der Anmeldung:
**21.05.86 Patentblatt 86/21**

(45) Bekanntmachung des Hinweises auf die
Patenterteilung:
**16.05.90 Patentblatt 90/20**

(84) Benannte Vertragsstaaten:
**AT BE CH DE FR GB IT LI LU NL SE**

(56) Entgegenhaltungen:
DE-A-2 750 207
FR-A-2 552 308
GB-A-1 178 294

CHEMICAL ABSTRACTS, Band 95, Nr. 18, 2.
November 1981, Seite 382, Nr. 156580m,
Columbus, Ohio, US; & JP - A - 81 46 837 (KOWA
PHARMACEUTICAL INDUSTRY CO., LTD.) 28-04-
1981

(73) Patentinhaber: **Gergely, Gerhard, Dr.**
**Gartengasse 8**
**A-1050 Wien (AT)**

(72) Erfinder: **Gergely, Gerhard, Dr.**
**Gartengasse 8**
**A-1050 Wien (AT)**
Erfinder: **Gergely, Thomas, Dr.**
**Gartengasse 8**
**A-1050 Wien (AT)**
Erfinder: **Gergely, Irmgard**
**Gartengasse 8**
**A-1050 Wien (AT)**

(74) Vertreter: **Büchel, Kurt F., Dr.**
**Bergstrasse 297**
**FL-9495 Triesen (LI)**

(56) Entgegenhaltungen:

CHEMICAL ABSTRACTS, Band 91, Nr. 10, 3.
September 1979, Seite 270, Nr. 78819x,
Columbus, Ohio, US; M. BARZEGAR-JALALI et
al.: "The effect of suspending agents on the
release of aspirin from aqueous suspensions in
vitro", & INT. J. PHARM. 1979, 2(3-4), 195-201
The theory and practice of Industrial Pharmacy
(2nd ed.), 1976 Lachmun et al, page 200
H.P. FIEDLER: "Lexikon der Hilfsstoffe", page
476, 1981

## Beschreibung

Die Erfindung betrifft eine pharmazeutische Zubereitung mit einem Gehalt an Ibuprofen, sowie ein Verfahren zu ihrer Herstellung.

Es ist bekannt, dass es Wirkstoffe gibt, die aus einer in Mund- und Rachenhöhle stark schleimhautreizenden Säure bestehen bzw. einen diesbezüglich stark reizenden Säurerest aufweisen. Ein derartiger, im übrigen schwer- bis unlöslicher Wirkstoff ist das Ibuprofen.

Ibuprofen ist ein Wirkstoff, der zur Rheuma- und Arthritisbekämpfung zunehmende Bedeutung gewonnen hat. Er ist in Wasser unlöslich, hat aber einen sehr unangenehmen Geschmack; insbesondere werden die Schleimhäute der Speiseröhre gereizt, offensichtlich durch die Zusammensetzung (2-(4-Isobutylphenyl)propionsäure). Ebenfalls aus der Literatur ist bekannt, dass Ibuprofen gastro-intestinales Bluten hervorrufen kann, ähnlich wie die Acetylsalicylsäure, insbesondere dann, wenn diese Substanz in höherer Konzentration, sei es in Form einer Tablette oder einer Kapsel, an die Magenwand gerät. Es ist daher ein therapeutischer Vorteil, wenn diese Substanz bereits vor Einnahme in Wasser suspendiert wird, so dass sich lokale Ueberkonzentrationen im gastro-intestinalen Trakt nicht mehr entwickeln können. Diese Suspendierung in Wasser ist aber aus geschmacklichen Gründen ohne zusätzliche Massnahmen nicht möglich, da Teile des Wirkstoffes im Mund und an der Speiseröhre hängenbleiben und dort zu kratzenden und reizenden Geschmackssensationen führen.

Der Erfindung liegt die Aufgabe zugrunde, eine pharmazeutische Zubereitung der eingangs genannten Art, sowie ein Verfahren zu deren Herstellung anzugeben, welche den vorstehend beschriebenen negativen geschmacklichen Effekt beseitigt und die problemlose orale Einnahme von Ibuprofenhaltigen Arzneimitteln gewährleistet.

Erfindungsgemäss wird diese Aufgabe bei einer pharmazeutischen Zubereitung der gattungsgemässen Art dadurch gelöst, dass die Ibuprofenpartikel mit einem Ueberzug aus wenigstens einem organischen Hydrokolloid und Fumarsäure umhüllt sind.

Dabei kann vorgesehen sein, dass das organische Hydrokolloid Xanthan und/oder Maltodextrin aufweist.

Weiterhin ist die erfindungsgemässe pharmazeutische Zubereitung gegebenenfalls gekennzeichnet durch einen Gehalt an einer Brausemischung.

Dabei kann vorgesehen sein, dass die Brausemischung Zitronensäure und Calciumcarbonat aufweist, wobei das Calciumcarbonat die Zitronensäure unter Haftvermittlung durch eine Bindeschicht umhüllt, die durch Anreaktion des Calciumcarbonates mit der oberflächennahen Schicht der Zitronensäurekristalle gebildet ist.

Das erfindungsgemäss vorgeschlagene Verfahren zum Herstellen einer pharmazeutischen Zubereitung der erfindungsgemässen Art ist dadurch gekennzeichnet, dass die Ibuprofenpartikel in einer Vakuummischmaschine unter Vakuum mit dem Ueberzug aus dem wenigstens einen organischen Hydrokolloid und Fumarsäure umhüllt und anschliessend vakuumgetrocknet werden.

Dabei kann insbesondere vorgesehen sein, dass zunächst das Ibuprofen, und das organische Hydrokolloid bzw. die organische Hydrokolloiden mit Wasser in der Vakuummischmaschine bei einem Druck von ca. 0,1 bar gemischt werden; und dass nach Antrocknen auf ca. 0,2 bar die Fumarsäure zugegeben wird, woraufhin das völlige Trocknen erfolgt.

Der Erfindung liegt die Erkenntnis zugrunde, dass der negative geschmackliche Effekt der bisherigen Ibuprofenpräparate dadurch beseitigt werden kann, dass man Ibuprofen mit organische Hydrokolloiden und Fumarsäure umhüllt; durch die Einhüllung der Ibuprofen-Kristalle in das organische Hydrokolloid bei gleichzeitiger Anwesenheit vom Fumarsäure, die einen niedrigeren pH-Wert aufweist, wird der negativen Geschmackseffekt verhindert; die organische Hydrokolloiden bewerkstelligen lediglich die Funktion der Verklammerung des wasserunlöslichen Ibuprofens mit der schwer löslichen Fumarsäure. Die Herstellung der erfindungsgemässen pharmazeutischen Zubereitung bzw. die Durchführung des erfindungsgemässen Verfahrens erfolgen dabei im übrigen zweckmässiger- und vorteilhafterweise mittels einer Vakuummischmaschine und mittels eines Verfahrens, wie es Gegenstand der DE—OS 3434774.7 ist, auf die zur ergänzenden Erläuterung des Erfindungsgedankens insoweit in vollem Umfang Bezug genommen wird.

Nachstehend ist die Erfindung anhand von Ausführungsbeispielen im einzelnen erläutert.

Beispiel 1

200 Teile Ibuprofen werden mit einer Lösung von 10 Teilen Xanthan und 10 Teilen Maltodextrin in 50 Teilen Wasser behandelt; vor dem völligen Trocknen in Vakuum werden bei ca. 200 mbar 20 Teile Fumarsäure zugesetzt. Nach dem völligen Trocknen entsteht ein Granulat, das auf eine Korngrösse von ca. 0,3 mm gemahlen wird.

Diese Teilchen können nun in einer Instanttechnologie auf Kristallzucker aufgebracht werden, wobei vorzugsweise die 10 bis 15-fache Menge Zucker angewandt wird und die doppelte Menge Zitronensäure hinzugefügt werden kann.

Stellt man ein derartiges Gemisch auf eine Einzeldosis von 200 mg Ibuprofen her, dann entsteht bei geeigneter Aromatisierung ein angenehmes Getränk, das keinerlei Sensationen auf die Schleimhäute mehr ausübt.

Beispiel 2

Dasselbe System, wie vorher geschildert, kann verbessert werden, indem man eine Brausemischung von Zitronensäure und Calciumcarbonat beifügt.

Man lässt 50 Teile Zitronensäure mit 50 Teilen Calciumcarbonat im Vakuum reagieren, indem man 5 Teile 50%iges Ethanol hinzufügt. Dieser Brauseteil wird getrocknet und in 5-facher Menge dem nach Beispiel 1 behandelten Ibuprofen hinzugefüft.

Ein so hergestelltes Produkt zeigt den selbstsuspendierenden Effekt einer langsamen Brausemischung, wobei offensichtlich die vorhandenen Calciumionen einen weiteren Verbesserungseffekt auf den Geschmack des Ibuprofen ausüben.

**Patentansprüche**

1. Pharmazeutische Zubereitung mit einem Gehalt an Ibuprofen, dadurch gekennzeichnet, dass die Ibuprofen-Partikel mit einem Ueberzug aus wenigstens einen organischen Hydrokolloid und Fumarsäre umhüllt sind.

2. Zubereitung nach Anspruch 1, dadurch gekennzeichnet, dass das organische Hydrokolloid Xanthan enthält.

3. Zubereitung nach Anspruch 1 oder 2, dadurch gekennzeichnet, dass das Organische Hydrokolloid Maltodextrin enthält.

4. Zubereitung nach einem der vorangehenden Ansprüche, gekennzeichnet durch einen Gehalt an einer Brausemischung.

5. Zubereitung nach Anspruch 4, dadurch gekennzeichnet, dass die Brausemischung Zitronensäure und Calciumcarbonat aufweist, wobei das Calciumcarbonat die Zitronensäure unter Haftvermittlung durch eine Bindeschicht umhüllt, die durch Anreaktion des Calciumcarbonates mit der oberflächennahen Schicht der Zitronensäurekristalle gebildet ist.

6. Verfahren zum Herstellen der pharmazeutischen Zubereitung nach einem der vorangehenden Ansprüche, dadurch gekennzeichnet, dass die Ibuprofen-Partikel in einer Vakuummischmaschine unter Vakuum mit dem Ueberzug aus dem wenigstens einen organischen Hydrokolloid und Fumarsäure umhüllt und anschliessend vakuumgetrocknet werden.

7. Verfahren nach Anspruch 6, dadurch gekennzeichnet, dass zunächst das Ibuprofen und das organische Hydrokolloide bzw. die organische Hydrokolloiden mit Wasser in der Vakuumischmaschine bei einem Druck von ca. 0,1 bar gemischt werden, und dass nach Antrocknen bis auf ca. 0,2 bar die Fumarsäure zugegeben wird, woraufhin das völlige Trocknen erfolgt.

**Revendications**

1. Préparation pharmaceutique contenant de l'ibuprofène caractérisée en ce que les particules d'ibuprofène sont enrobées d'un revêtement constitué d'au moins un hydro-colloïde organique et d'acide fumarique.

2. Préparation selon la revendication 1, caractérisée en ce que l'hydro-colloïde organique contient du xanthane.

3. Préparation selon la revendication 1 ou 2, caractérisée en ce que l'hydro-colloïde organique contient de la maltodextrine.

4. Préparation selon l'une des revendications précédentes, caractérisée en ce qu'elle contient un mélange effervescent.

5. Préparation selon la revendication 4, caractérisée en ce que le mélange effervescent comporte de l'acide citrique et du carbonate de calcium, le carbonate de calcium enveloppant l'acide citrique avec adhérence par une couche de liant, qui est formée par l'amorce de réaction du carbonate de calcium avec la couche proche de la surface des cristaux d'acide citrique.

6. Procédé pour la fabrication de la préparation pharmaceutique selon l'une des revendications précédentes, caractérisé en ce que les particules d'ibuprofène sont enrobées sous vide de la couche comportant au moins un hydro-colloïde organique et de l'acide fumarique dans un mélangeur sous vide et ensuite séchées sous vide.

7. Procédé selon la revendication 6, caractérisé en ce que l'on mélange d'abord d'ibuprofène et l'hydro-colloïde organique ou les hydro-colloïdes organiques avec de l'eau dans le mélangeur sous vide sous une pression d'environ 0,1 bar et en ce qu'après un début de séchage on ajoute l'acide fumarique jusqu'à environ 0,2 bars, le séchage complet étant effectué ensuite.

**Claims**

1. An ibuprofen containing pharmaceutical preparation, characterized in that the ibuprofen particles are covered with a coating of at least one organic hydro-colloid and of fumaric acid.

2. A pharmaceutical preparation as in claim 1, characterized in that the organic hydro-colloid contains xanthan.

3. A pharmaceutical preparation as in claims 1 or 2, characterized in the organic hydro-colloid contains maltodextrin.

4. A pharmaceutical preparation as in one of the above claims, characterized in that it comprises an effervescent mixture.

5. A pharmaceutical preparation as in claim 4, characterized in that the effervescent mixture comprises citric acid and calcium carbonate, whereby the calcium carbonate envelops the citric acid by means of a bonding layer acting as a coupling agent, that is formed by the reaction of the calcium carbonate with the surface adjacent layer of the citric acid crystals.

6. A process for manufacture of an ibuprofen containing pharmaceutical preparation as in one of the above claims, characterized in that the ibuprofen particles are enveloped with a coating of at least one organic hydro-colloid and of fumaric acid under vacuum conditions, and that the resulting mixture is vacuum dried.

7. A process in accordance with claim 6, characterized in that the ibuprofen and the organic hydro-colloid on the organic hydro-colloids are mixed with water in the vacuum mixing machine under a pressure of about 0.1 bar and that after that this mixture is partially dried to a pressure of about 0.2 bar fumaric acid is added to this mixture, and that the resulting mixture is dried to completion.